# EUROPEAN PATENT APPLICATION

(11) **EP 0 652 195 A1**
(43) Date of publication of application: **10.05.1995**
(21) Application number: 94203136.0
(22) Date of filing: 27.10.1994
(51) Int. Cl.: C07C 17/00, C07C 19/04

(54) **Preparation of CHC13 by starting from CC14**

(30) Priority: 04.11.1993 IT MI932333
(71) Applicant: ENICHEM S.p.A., I-20124 Milano (IT)
(72) Inventor: Nurra, Carmelo, IT-07040 Olmedo (Sassari) (IT); Mansani, Riccardo, IT-07100 Sassari (IT); Cappellazzo, Oscar, IT-07041 Alghero (Sassari) (IT); Cesana, Alberto, IT-20048 Carate Brianza (Milano) (IT)
(74) Representative: Fusina, Gerolamo

(57) **Abstract**

CHCl₃ is produced by catalytic hydrogenation of CCl₄ in the liquid phase, with a supported Pt or Pd catalyst, and in the presence of at least one solvent.

The reduction reaction can be carried our both continuously and batchwise and can be applied to the industrial production of CHCl₃.

## Description

The present invention relates to a process for producing chloroform by starting from carbon tetrachloride; the process is based on the catalytic reduction of CCl₄ in the liquid phase, with supported Pt or Pd catalysts and in the presence of at least one solvent.

The reduction reaction can be carried out both continuously and batchwise and can be applied to the industrial production of CHCl₃; it is carried out in the presence of suitable catalysts and using pure hydrogen at temperatures comprised within the range of from a minimal value of 100°C up to > 160°C, with the pressure values being comprised within the range of from 10 to 50 atmospheres.

The present invention also relates to chloroform obtained according to the process of the present invention.

The reaction of reduction of carbon tetrachloride to chloroform is known from the prior art; it was not very widely used heretofore, owing to the poor availability of CCl₄, largely used in CFC's synthesis.

However, the recent limitations to use of CFC's, imposed owing to the recognized effect of depletion of the stratospheric ozone layer caused by them, led to a very large availability of CCl₄ on the market, and to the consequent search for alternative usages; among them, the use in chloroform production was the most deeply studied one.

In general, the methods proposed by the present state of the art are based on the reaction of CCl₄ reduction with hydrogen in the absence of solvents; more generally, the reactions carried out in gas phase or liquid phase are known, in which the liquid phase consists of the same CCl₄.

However, all processes known from the prior art are based on catalytic hydrogenation reaction of CCl₄, as schematically shown as follows:

CCl₄ + H₂ ----cat.----> CHCl₃ + HCl

From the various examples of liquid phase reaction of carbon tetrachloride, we remind EP 0 460 138 and Japanese patent application JP 91/119,457, and, as examples of gas phase reaction, we remind US 2,886,605 which discloses a process using a catalyst based on copper halide on fluidized bed; US 3,579,596 to DOW Chem., using a Pd based catalyst on fixed bed and EP 0 486 091 to Solvay, which discloses a gas phase process using methane as diluent.

All the proposed systems which, as said, operate in gas phase or in liquid phase in the absence of solvents, respectively display two main types of drawbacks:
(a) They coproduce large amounts of methane (Solvay, Atochem), causing an increase in hydrogen consumption;
(b) They coproduce chlorinated C₂ compounds, such as perchloroethylene (Ercros, Ag Technology Co) which, by being subject to undergo polymerization, expose the catalyst to higher fouling risks.

Furthermore, as regards the prior art, one may observe the following: the systems operating in the gas phase display, with the reactor size being the same, a reduced productivity as compared to systems operating in the liquid phase, owing to the higher rarefaction of reactant streams; on the other hand, the processes operating in the liquid phase display, for such exothermic reactions as those taken into consideration, considerable problems of reactor temperature control (in the case of the reduction of CCl₄ to CHCl₃ at 140°C, an exotherm of -24 Kcal/mol may be observed). In these cases, the presence of a solvent which can disperse the generated heat through a larger reaction mass, appears to be the choice system to overcome these problems.

The presence of a solvent may furthermore perform a favourable effect on catalyst life, by contributing to dissolve possible species deriving from byproduct polymerization which, if not properly removed in due time, might cause phenomena of deactivation by fouling.

Notwithstanding the above experiences, which should promote the use of solvents in the reaction of reduction of CCl₄ with hydrogen, the proposed processes heretofore are, as mentioned above, all directed to reactions not using any solvents.

The present Applicant found now that chloroform can be produced by hydrogenating CCl₄ in the presence of a solvent, a suitable catalytic system, with simultaneously preserving the typical conversion and selectivity yields of the prior art. The proposed process can be carried out both continuously and batchwise and makes it possible the amounts of such secondary products, as methane and chlorinated C₂ hydrocarbons (essentially C₂Cl₄ and C₂Cl₆) to be considerably reduced as compared to when the process is carried out under the same conditions, in the absence of solvent.

Therefore, the purpose of the present invention is a process for producing chloroform, which consists in bringing carbon tetrachloride into contact with hydrogen in the presence of a catalyst constituted by supported platinum and/or palladium, with the process being characterized in that the reaction is carried out in the liquid phase and in the presence of a solvent.

As the solvent, at least one from those hydrocarbons is used, which belong to the group comprising: pentane, hexane, eptane, octane, isooctane, decane, and so forth, or to the group comprising benzene, toluene, xylene, or the same species substitued with one or more linear or branched aliphatic chains.

The use is also possible of other stable solvents under the reaction conditions (e.g., flourinated and/or chlorinated hydrocarbons), as well as of mixtures of two or more solvents among those listed above.

The concentration of carbon tetrachloride in the reaction solution, constituted by the same carbon tetrachloride and the solvent, is generally comprised within the range of from 10 to 80% by weight; such a concentration is preferably comprised within the range of from 30 to 40% by weight.

The reduction reaction can be carried out both continuously and batchwise, and can be applied to the industrial production of CHCl₃; it is carried out by using neat hydrogen at temperatures comprised within the range of from a minimal value of 100°C up to 180°C, with the reaction pressure being comprised within the range of from 10 to 50 atmospheres. The reactions carried out in an aliphatic hydrocarbon solvent at temperatures comprised within the range of from 110 to 150°C and under pressures comprised within the range of from 20 to 40 atmospheres, are preferred.

As said, carbon tetrachloride is a commercial product which is largely available from the market, like hydrogen and the catalytic systems envisaged. As regards the catalytic systems, those in which the noble metal is supported on a carbon carrier are preferred, even if other types of supports can be used with good results as well.

The following examples are supplied in order to illustrate the present invention without limiting it.

### Example 1 (comparison example)

To an autoclave of Hastelloy, of 300 cm³ of capacity, 15.2 g of CCl₄ and 0.8 g of the commercial catalyst Engelhard 40122, constituted by 0.5% Pd on carbon scales, previously reduced to a powder, are charged.

After purging the autoclave with nitrogen and venting it, hydrogen is charged at room temperature until a pressure of 19 atmospheres, corresponding to a molar ratio of H₂/CCl₄ = 2.2 is reached.

The magnetically stirred autoclave is then immersed in a hot oil bath, and the internal autoclave temperature and pressure reach, within a short time, the values of 140°C and 27 atm, respectively.

After a residence time inside the bath of 4 hours and fast cooling of the system, a liquid phase and a gas phase are recovered which, when characterized by gas chromatography, display, in the gas phase, the presence of HCl, methane and ethane; and in the liquid phase, besides unreacted CCl₄, CHCl₃, C₂Cl₄, C₂Cl₆, C₄Cl₆, and unidentified products.

CCl₄ conversion results to be of 51.9% by weight, and the selectivity to CHCl₃ is of 19.3%.

### Example 2

In a test run according as disclosed in Example 1, 15.65 g of CCl₄, 29.2 g of n-octane and 0.8 g of the same commercial catalyst Engelhard 40122 are charged.

The conditions for reactants charging and test run execution are the same as of Example 1.

At the end of the test run, a gas phase and a liquid phase are recovered which contain HCl, methane and ethane and, respectively, besides n-octane and unreacted CCl₄, also CHCl₃, C₂Cl₄ and C₂Cl₆, together with some unidentified products.

The conversion of CCl₄ results to be of 49.8% by weight, and the selectivity to CHCl₃ of 49.2%.

### Example 3

In a test run similar to as disclosed in Example 1, however carried out at the temperature of 153°C and under the autogenous system pressure (9.9 atm), 15.51 g of CCl₄, 29.0 g of benzene and 0.8 g of commercial catalyst Engelhard 40122 are charged.

At the end of the test run, the usual gas and liquid phases are recovered, which contain, respectively: HCl, methane, ethane and unreacted CCl₄ in the gas phase; and benzene, CHCl₃, CCl₄, C₂Cl₄ in the liquid phase.

The conversion of CCl₄ is of 47.5% by weight, and the selectivity to CHCl₃ is of 23.6%.

### Example 4

In a test run carried out as disclosed in Example 1, 15.5 g of CCl₄ and 0.39 g of commercial catalyst Engelhard 40150, constituted by 1% platinum, by weight, on carbon powder are charged.

At the end of the test run, both the gas and liquid phases are recovered; the gas phase contains: HCl, methane, ethane and unreacted CCl₄; the liquid phase contains: CHCl₃, C₂Cl₄, C₂Cl₆, C₄Cl₆, besides unidentified products.

CCl₄ conversion results to be of 99.5%, and the selectivity to CHCl₃ is of 28.6%.

### Example 5

In a test run as disclosed in Example 1, 15.62 g of CCl₄, 29.5 g of a paraffin blend constituted by a C₁₀-C₁₃ cut of linear paraffins and 0.39 g of commercial catalyst constituted by 1% Pt on carbon powder, are charged.

At the end of the test run, both phases are recovered, as usual; the gas phase contains: HCl, methane, ethane; the liquid phase contains: unreacted CCl₄, CHCl₃, C₂Cl₄, C₂Cl₆, C₄Cl₆, together with the paraffinic blend and unidentified products.

CCl₄ conversion results to be of 99.4%, and the selectivity to CHCl₃ of 76%.

### Example 6

In a test run as disclosed in Example 1, 15.5. g of CCl₄, 30.2 g of benzene and 0.39 g of commercial catalyst Engelhard 40159, constituted by 1% Pt on carbon powder, are charged.

Both phases recovered at test run end respectively contain: the gas phase HCl, methane, ethane; and the liquid phase: unreacted CCl₄, CHCl₃, C₂Cl₄, C₄Cl₆, benzene, and unidentified products.

The conversion of CCl₄ is of 47.6, the selectivity to CHCl₃ of 54.4%.

### Example 7

A mixture constituted by CCl₄ (35% by weight) and n-paraffins (C₁₀-C₁₃ cut of linear paraffins), is fed, at the flow rate of 670 g/h, to a tubular reactor of Hastelloy C (inner diameter 15.8 mm; length 1.7 m; total volume 343 cm³), containing 77.4 cm³ (50 g) of the commercial catalyst SUD-CHEMIE AG-M-220, constituted by 0.2% platinum supported on gamma-alumina carrier, at the temperature of 120°C and under a pressure of 30 atm. The reaction effluent, at room temperature, is constituted by two phases, i.e., a gas phase and a liquid phase, which are individually analyzed by chromatography.

The test run yielded a conversion of CCl₄ of 30%, with a selectivity to CHCl₃ of 80%.

The byproducts are essentially constituted by methane (selectivity 15%), present in the gas phase, and C₂Cl₆ (selectivity 1%), present in the liquid phase.

The situation reported hereinabove remains unchanged during the 20-hour test run of the reactor.

The results of the above experimental tests (Examples 1-7) clearly demonstrate the favourable effect of the presence of a solvent in the reaction environment when CCl₄ hydrogenation to yield CHCl₃ is carried out batchwise. On the other hand, the so obtained advantages, essentially consisting in the marked increase in selectivity to CHCl₃, are retained (see Example 7) when the process is carried out continuously, in such a way configuring the possibility of application of the finding at an industrial level.

Yields and selectivities, obtained with the catalysts used by the Applicant, in the systems according to the present invention, result to be comparable to those known from the prior art. However, the phenomenon we detected appears to be of general character and is suitable for improving the results which can be obtained in any systems (either batchwise or continuous), with any catalysts. Moreover, the use of a solvent may enable an industrial process to be developed which, from the thermal view point is more controllable as compared to a conventional process in the liquid phase, and allows a higher productivity to be accomplished than the gas phase processes.

## Claims

1. Process for producing chloroform, which consists in bringing into contact, under suitable conditions to cause a reduction reaction to take place, carbon tetrachloride with hydrogen in the presence of a catalyst constituted by supported platinum and/or palladium, said process being characterized in that the reaction is carried out in the liquid phase and in the presence of a solvent.

2. Process according to claim 1, characterized in that the concentration of carbon tetrachloride in the reaction solution, constituted by same carbon tetrachloride and the solvent, is comprised within the range of from 10 to 80% by weight; preferably, such a concentration is comprised within the range of from 30 to 40% by weight.

3. Process according to claim 1, characterized in that the reaction is carried out at a temperature comprised within the range of from 100 to 180°C and under a pressure comprised within the range of from 10 to 50 atm.

4. Process according to claim 1, characterized in that the reaction is carried out in an aliphatic hydrocarbon solvent, at a temperature comprised within the range of from 130 to 150°C and under a pressure comprised within the range of from 20 to 40 atm.

5. Process according to claim 1, characterized in that as solvent at least one from the hydrocarbons is used, which belong to the group comprising: pentane, hexane, heptane, octane, isooctane, decane, and so forth, or to the group comprising benzene, toluene, xylene or substituted derivatives of the same products, with one or more either linear or branched aliphatic chains.

6. Process according to claim 1, characterized in that as the solvent a fluorinated and/or chlorinated hydrocarbon is used.

7. Chloroform, obtained according to the process of claims from 1 to 5.
